# EUROPEAN PATENT SPECIFICATION

(11) **EP 1 931 364 B1**
(45) Date of publication and mention of the grant of the patent: **21.07.2010**
(21) Application number: 06806096.1
(22) Date of filing: 06.10.2006
(51) Int. Cl.: A61K 35/74, A23K 1/00

(54) **PROBIOTIC ENTEROCOCCI FOR IMPROVED IMMUNITY**
PROBIOTISCHE ENTEROKOKKEN FÜR EINE VERBESSERTE IMMUNITÄT
ENTEROCOQUES PROBIOTIQUES PERMETTANT D'AMELIORER L'IMMUNITE

(30) Priority: 06.10.2005 US 724214 P
(43) Date of publication of application: 18.06.2008
(73) Proprietor: Nestec S.A., 1800 Vevey (CH)
(72) Inventor: KNORR, Ruth, 80800 Bussy-les-Daours (FR); CAVADINI, Christoph, 1804 Corsiers-sur-Vervey (CH); BENYACOUB, Jalil, 1010 Lausanne (CH); SATYARAJ, Ebenezer, Wildwood, MO 63040 (US)
(74) Representative: Lock, Graham James
(86) International application number: PCT/EP2006/009695
(87) International publication number: WO 2007/039313

(56) References cited:
- EP-A- 1 364 586
- WO-A-01/90311
- WO-A-98/00035
- BENYACOUB J ET AL: "Enterococcus faecium SF68 enhances the immune response to Giardia intestinalis in mice." THE JOURNAL OF NUTRITION MAY 2005, vol. 135, no. 5, May 2005 (2005-05), pages 1171-1176, XP002415804 ISSN: 0022-3166
- BENYACOUB JALIL ET AL: "Supplementation of food with Enterococcus faecium (SF68) stimulates immune functions in young dogs." THE JOURNAL OF NUTRITION APR 2003, vol. 133, no. 4, April 2003 (2003-04), pages 1158-1162, XP002415805 ISSN: 0022-3166
- WEESE J SCOTT ET AL: "Bacteriological evaluation of dog and cat diets that claim to contain probiotics." THE CANADIAN VETERINARY JOURNAL. LA REVUE VÉTÉRINAIRE CANADIENNE MAR 2003, vol. 44, no. 3, March 2003 (2003-03), pages 212-216, XP002415806 ISSN: 0008-5286
- BIERMAN H R ET AL: "Remissions in leukemia of childhood following acute infectious disease: staphylococcus and streptococcus, varicella, and feline panleukopenia." CANCER MAY 1953, vol. 6, no. 3, May 1953 (1953-05), pages 591-605, XP002415807 ISSN: 0008-543X
- DE VRESE M ET AL: "Effect of Lactobacillus gasseri PA 16/8, Bifidobacterium longum SP 07/3, B. bifidum MF 20/5 on common cold episodes: A double blind, randomized, controlled trial" CLINICAL NUTRITION, CHURCHILL LIVINGSTONE, LONDON, GB, vol. 24, no. 4, August 2005 (2005-08), pages 481-491, XP004996029 ISSN: 0261-5614
- GILL H S ET AL: "Enhancement of immunity in the elderly by dietary supplementation with the probiotic Bifidobacterium lactis HN019." THE AMERICAN JOURNAL OF CLINICAL NUTRITION DEC 2001, vol. 74, no. 6, December 2001 (2001-12), pages 833-839, XP002415808 ISSN: 0002-9165

## Description

### FIELD OF THE INVENTION

The present invention is related to mammalian nutrition and effects thereof on the immune response. In particular, the present invention utilizes probiotics organisms, administered orally to a feline animal, to improve both innate and adaptive immunity and to enhance vaccine efficacy in the animal.

### BACKGROUND OF THE INVENTION

Various publications, including patents, published applications, technical articles and scholarly articles are cited throughout the specification. Full citations for publications not cited fully within the specification are set forth at the end of the specification.

Probiotics have been defined as live microorganisms that, when administered in adequate amounts, confer a health effect on the host. (Schrezenmeir J *et al.* (2001)). It is theorized that probiotics may impart their beneficial health effects either by increasing the resistance to colonization of mucosal surfaces by pathogenic bacteria (colonization resistance) (Sanders ME (2003)) or by exerting a direct effect on gut associated lymphoid tissue (GALT), resulting in the production of immunomodulating substances. (Isolauri E *et al*. (2001 a); and Macpherson AJ *et al.* (2004)).

Probiotics have been used to modulate the course of a variety of infectious diseases in human medicine. (Isolauri E (2001b)). In contrast, few studies have been performed in veterinary medicine, with the majority of veterinary studies being in large animals, where probiotics have been used to attempt to alter the shedding of fecal pathogens (Kim LM *et al.* (2001)) or to improve production parameters such as weight gain, feed conversion rate and reduced mortality. In one animal study, *Enterococcus faecium* strain SF68 (NCIMB10415) was fed to a group of puppies vaccinated with canine distemper virus (CDV) and compared to a control group that received vaccinations only. (Benyacoub J *et al.* (2003)). Puppies supplemented with SF68 had increased serum and fecal total IgA concentrations, increased CDV-specific IgG and IgA serum concentrations, and increased percentage of circulating B lymphocytes compared to control puppies proving an immune enhancing effect induced by this probiotic.

Feline panleukopenia (FPV) is a virus resulting in viremia followed by severe gastrointestinal disease; appropriately vaccinated kittens have sterilizing immunity. (Richards J *el al*. (2001)). However, viral upper respiratory tract infections continue to be a major problem in feline medicine. (Sykes JE et al. (1999)). Feline rhinotracheitis (FHV-1) and feline calicivirus (FCV) are the two viral pathogens implicated in the syndrome. While FCV vaccines induce > 95% relative efficacy in vaccinates when compared to unvaccinated controls after being inoculated with a pathogenic challenge strain, FHV-1 vaccines only induce approximately 60% relative efficacy. (Lappin MR *et al.* 2002)). Thus, FHV-1 continues to be a significant problem despite widespread vaccination. (Sykes JE *et al.* (1999)). Previous attempts at improving efficacy of vaccination have included intranasal administration, which leads to greater side effects, (Scott FW *et al.* (1999)) and genetic manipulation of virulent strains, which leads to decreased disease severity but does not decrease the prevalence of the carrier state. (Slater E *et al.* (1976)) The carrier state can lead to recrudescence or reinfection of the host as well as transmission to housemates. Multiple therapies for chronic FHV-1 infections have been tried, including interferon alpha, trephination, antiviral drugs, rhinotomy, glucocorticoids, topical decongestants, and antibiotics directed at secondary bacterial infections. (Van Pelt DR *et al.* (1994)). However, none of these has been able to clear the chronic viral infection; therefore recurrences of viral shedding and clinical illness are common. Both cell-mediated and IgA mucosal immune responses are considered important in prevention and control of α-herpesvirus infections. (Lappin MR *et al.* (2002); and Slater E *et al.* (1976)). Improved FHV-1 vaccines or responses to vaccinations are needed to lessen morbidity induced by this pathogen.

### SUMMARY OF THE INVENTION

It will be appreciated that the scope of protection is as defined in the appended claims.

In one aspect of the present invention, there is provided use of a probiotic *Enterococcus* bacteria, administered orally, in the preparation of a medicament for enhancing the vaccine efficacy of a FVH-1, FCV or FPV vaccine in a feline animal.

In another aspect, there is provided a composition comprising one or more probiotic *Enterococcus* bacteria, administered orally, for enhancing the vaccine efficacy of a FVH-1, FCV or FPV vaccine in a feline animal.

Modulating the immune response and enhancing vaccine efficacy serve to protect the animal and lessen morbidity and mortality induced by pathogens.

In certain embodiments, the composition is a pet or animal food composition or dietary supplement. In various embodiments, the probiotic organisms include at least one of *Enterococcus spp*., alone or combined with other probiotic organisms, such as *Streptococcus spp., Lactobacillus spp., Lactococcus spp., Bacillus spp., Bifidobacterium spp., or Saccharomyces spp*. In preferred embodiments, the probiotic organism is *Enterococcus faecium* NCIMB 10415 (SF68). The compositions may comprise additional ingredients. For example, one or more compounds that further enhance immunity such as 7-oxo dehyroepiandrosterone are included.

In certain embodiments, the compositions are formulated for companion animals, such as a cat. In other embodiments, the compositions are formulated for non-companion animals, particularly for members of the cat family.

Also described is a method for modulating immunity in an animal, comprising administering to the animal on a regular basis a composition comprising one or more probiotic organisms, as described above, in an amount effective to modulate immunity in the animal. In some examples, the method is applied to a companion animal, such as a cat. In other embodiments, the method is applied to non-companion animals, particularly members of the cat family.

Further described is a method for enhancing vaccine efficacy in an animal, comprising administering to the animal on a regular basis a composition comprising one or more probiotic organisms, as described above, in an amount effective to enhance vaccine efficacy in the animal. In some examples, the vaccine is to feline rhinotracheitis virus, feline calcivirus, or feline panleukopenia virus. In some examples, the method is applied to a companion animal, such as a cat. In other examples, the method is applied to non-companion animals, particularly members of the cat family.

Other features and advantages of the invention will become apparent by reference to the drawings, detailed description and examples that follow.

### BRIEF DESCRIPTION OF THE DRAWINGS

**Figure 1****.** Body weights (a) and fecal scores (b) over time of kittens supplemented with 150 mg chicken digest PO (Placebo, n=9) or 150 mg chicken digest mixed with 5 x 10⁸ cfu/day *Enterococcus faecium* strain SF68 (Treatment, n=9) daily starting at 7 weeks of age until 27 weeks of age. Kittens were vaccinated subcutaneously with a commercially available, modified live FHV-1 vaccine^{d} at 9 and 12 weeks of age. Box and whiskers represent the minimum, maximum, median and 25th and 75th percentiles. p>0.05 at all time points.
**Figure 2**. FHV-1 specific IgA results in serum (a) and saliva (b) from kittens with (Treatment) or without (Placebo) SF68 supplementation. Box and whiskers represent the minimum, maximum, median and 25th and 75th percentiles. p>0.05 for all time points
**Figure 3**. FHV-1 specific IgG results in serum from kittens with (Treatment) or without (Placebo) SF68 supplementation. Box and whiskers represent the minimum, maximum, median and 25th and 75th percentiles.p>0.05 for all time points.
**Figure 4****.** FCV specific IgG results from kittens with (Treatment) or without (Placebo) SF68 supplementation. Box and whiskers represent the minimum, maximum, median and 25th and 75th percentiles. p>0.05 for all time points.
**Figure 5****.** FPV specific IgG results from kittens with (Treatment) or without (Placebo) SF68 supplementation. Box and whiskers represent the minimum, maximum, median and 25th and 75th percentiles. p>0.05 for all time points.
**Figure 6****.** Total IgG (a) and IgA (b) in fecal extracts from kittens with (Treatment) or without (Placebo) SF68 supplementation. Box and whiskers represent the minimum, maximum, median and 25th and 75th percentiles. p>0.05 for all time points.
**Figure 7****.** Percent of gated lymphocytes positive for CD4 (a) and CD8 (b) in peripheral blood by flow cytometry in kittens with (Treatment) or without (Placebo) SF68 supplementation. Box and whiskers represent the minimum, maximum, median and 25th and 75th percentiles. * denotes time points at which treatment group was significantly higher than placebo group.

### DETAILED DESCRIPTION OF ILLUSTRATIVE EMBODIMENTS

### Definitions:

Various terms relating to aspects of the present invention are used throughout the specification and claims. Such terms are to be given their ordinary meaning in the art unless otherwise indicated. Other specifically defined terms are to be construed in a manner consistent with the definition provided herein.

The following abbreviations may be used in the specification and examples: FHV-1, feline rhinotracheitis virus; FCV, feline calcivirus; FPV, feline panleukopenia virus; spp., species; ELISA, enzyme linked immunosorbent assay; DM, dry matter; CFU, colony forming units; kg, kilogram; BW, body weight.

"Effective amount" refers to an amount of a compound, material, or composition, as described herein that is effective to achieve a particular biological result. Such results include, but are not limited to, improving immunity or enhancing vaccine efficacy in an animal. Such effective activity may be achieved, for example, by administering the compositions of the present invention to the animal.

Within the context of this specification the term "about" is interpreted to mean optionally plus or minus 20%, more preferably optionally plus or minus 10%, even more preferably optionally plus or minus 5%, even more preferably optionally plus or minus 2%, most preferably optionally plus or minus 1%.

"Comprising..." is interpreted to mean "includes, amongst other things..." and is not intended to mean "consisting of only...".

"Adaptive immunity" or "adaptive immune response" are used interchangeably and in a broad sense herein, and refer to the immune response to antigen challenge, including the development of immunological memory. The adaptive immune response includes, without limitation, humoral and cellular immunity.

"Humoral immunity" or "humoral immune response" are used interchangeably herein, and refer to the production of immunoglobulin molecules in response to an antigen challenge.

"Cellular immunity" or "cellular immune response" or "cell mediated immunity" are used interchangeably herein, and refer to the activation of cytotoxic or helper T-lymphocytes, mononuclear cells, and cytokines in response to an antigen challenge. The term encompasses all adaptive immunity that cannot be transferred to a naïve recipient with antibodies.

"Innate immunity" refers to the body's non-specific mechanisms for resistance to pathogens that are not enhanced upon subsequent challenge with a particular antigen.

"Modulate immunity" or "modulation of immunity" refers to any enhancement or inhibition of the body's ability to generate an innate or adaptive immune response to antigen challenge, as measured by any means suitable in the art.

"Vaccine efficacy" means the ability of a vaccine to produce a desired therapeutic or protective effect on an animal against a specified pathogen. "Enhanced vaccine efficacy" refers to any improvement in the ability of a vaccine to produce a desired therapeutic or protective effect on an animal against a specified pathogen, as measured by any means suitable in the art.

"Probiotic organism" refers to any organism, particularly microorganisms, that exert a beneficial effect on the host animal such as increased health or resistance to disease. Probiotic organisms can exhibit one or more of the following non-limiting characteristics: non-pathogenic or non-toxic to the host; are present as viable cells, preferably in large numbers; capable of survival, metabolism, and persistence in the gut environment (*e*.*g*., resistance to low pH and gastrointestinal acids and secretions); adherence to epithelial cells, particularly the epithelial cells of the gastrointestinal tract; microbicidal or microbistatic activity or effect toward pathogenic bacteria; anticarcinogenic activity; immune modulation activity, particularly immune enhancement; modulatory activity toward the endogenous flora; enhanced urogenital tract health; antiseptic activity in or around wounds and enhanced would healing; reduction in diarrhea; reduction in allergic reactions; reduction in neonatal necrotizing enterocolitis; reduction in inflammatory bowel disease; and reduction in intestinal permeability. (Reid G *et al.* (2003); Drisko JA *et al.* (2003); and Oyetayo VO *et al.* (2004)).

A "companion animal" is any domesticated animal, and includes, without limitation, cats, dogs, rabbits, guinea pigs, ferrets, hamsters, mice, gerbils, horses, cows, goats, sheep, donkeys, pigs, and the like. Cats are exemplified herein. In some examples, the "animal" may be a human. In another example, the invention relates to animals other than companion animals.
The invention relates to members of the Felidae, the cat family, to which the invention may be applied in instances where the cat is available to receive administration of the probiotic composition (*e*.*g*., in a zoo, veterinary facility, game preserve, and the like). In addition to the domestic cat, *Felis cattus*, the Felidae include members of the genera: (1) *Acinonyx,* such as the cheetah (*A. jubatus)*, (2) *Neofelis,* such as the clouded leopard (*N. nebulosa*), (3) *Panthera*, such as the lion (*P. leo*), jaguar (*P. onca*), leopard (P. pardus), tiger (P. tigris); (3) *Uncia,* such as the snow leopard (*U. uncial*); (4) *Puma,* such as the cougar, mountain lion or puma (*P. concolor*) and (5) various species of non-domesticated cats (*Felis*), including but not limited to Bomean bay cat (*F. badia*), Caracal (*F. caracal*), Chinese mountain cat (*F*. *bieti*), jungle cat (*F. chaus*), sand cat (*F. margarita*)*,* black-footed cat (*F. nigripes*), wildcats (*F. sylvestris, F. lybica*), jaguarondi (*F. yagouraroundi*), ocelot (*F. pardalis*), oncilla (*F. tigrina*)*,* margay (*F. wieldi*), serval (*F. serval*)*,* lynx (*F. lynx*), bobcat (*F. rufus*), pampas cat (*F. colocolo*), Geoffroy's cat (*F*. *geoffroyi*), Andean mountain cat (*F. jacobita*)*,* pallas cat (*F*. *manul*)*,* kodkod (*F*. *guigna*), leopard cat (*F*. *bengalensis, F. iriomotensis*), flat-headed cat (*F*. *planiceps*), rusty-spotted cat (*F. rubiginosus*), fishing cat (*F. viverrina*), and African golden cat (*F. aurata*). As used herein, the term "feline" or "feline animal" refers to all members of the cat family, unless specified otherwise.

As used herein, the term "pet food," "pet food composition," "animal food" or "animal food composition" means a composition that is intended for ingestion by an animal, and preferably by companion animals. A "complete and nutritionally balanced pet or animal food," is one that contains all known required nutrients in appropriate amounts and proportions based on recommendations of recognized authorities in the field of animal nutrition, and is therefore capable of serving as a sole source of dietary intake to maintain life or promote production, without the addition of supplemental nutritional sources. Nutritionally balanced pet food and animal food compositions are widely known and widely used in the art.

As used herein, a "dietary supplement" is a product that is intended to be ingested in addition to the normal diet of an animal.

As used herein, a "food product formulated for human consumption" is any composition intended for ingestion by a human being.

For the purposes or writing a clear and concise specification, certain embodiments have been described herein, but it will be appreciated that features of those embodiments can be variously combined or separated within the scope of the invention.

### Description:

The inventors have observed that dietary supplementation with probiotic organisms such as *Enterococcus faecium* NCIMB 10415 (SF68) in kittens increased the number of CD4+ lymphocytes. Accordingly, various aspects of the present invention utilize these discoveries by providing dietary compositions enhance vaccine efficacy in the animal.

### Compositions

One aspect of the invention features compositions comprising one or more probiotic *Enterococcus* bacteria, administered orally, for enhancing the vaccine efficacy of a FVH-1, FCV or FPV vaccine in a feline animal.

The probiotic organisms can be present in the composition as an ingredient or additive. The probiotic organisms can be prokaryotes, eukaryotes, or archaebacteria. In various embodiments of the composition, the probiotic organisms comprise at least one of any suitable strain or subspecies of *Enterococcus,* alone, or in combination with other probiotic organisms, included within such genera as *Streptocococcus., Lactobacillus., Lactococcus., Bacillus, Bifidobacterium, or Saccharomyce. Enterococcus* species include, without limitation, *Enterococcus facecium, specifically E. faecium* strain SF68, as well as other Enterococci such as *E. faecium* DSM 10663 (M74), *E. faecium* GHR 017 DSM 7134, *E. faecium* CECT 4515, *E. faecium* CL15/ATCC 19434, *E. faecium* NCIMB 11181/DSM 5464, *E. faecium* IMB 52/DSM 3530, *E. faecium* CNCM MA 17/5U, *E. faecium* 202 DSM 4788/ATCC53519, *E. faecium* 301 DSM 4789/ATCC 55593, *E. faecium* ATCC 19434, *E. faecium* EF-101 ATCC 19434, and *E. faecium* AK 2205 BCCM/LMG S-16555 *Streptococcus* species include, without limitation, *Streptococcus faecium, Streptococcus thermophilus,* and *Streptococcus salivarus. Lactobacillus* species include, without limitation, *Lactobacillus acidophilus. Lactobacillus brevis, Lactobacillus bulgaricus, Lactobacillus casei*, *Lactobacillus cellobiosus, Lactobacillus crispatus, Lactobacillus curvatus, Lactobacillus fermentum, Lactobacillus GG* (*Lactobticillus rhamnosus* or *Lactobacillus casei* subspecies *rhamnosus*), *Lactobacillus gasseri, Lactobacillus johnsonii, Lactobacillus plantarum, Lactobacillus salivarus, Lactobacillus reuteri, Lactobacillus johnsonii* LA1, *Lactobacillus acidophilus* NCFB 1748, *Lactobacillus casei Shirota, Lactobacillus acidophilus* NCFM, *Lactobacillus acidophilus* DDS-1, *Lactobacillus delbrueckii* subspecies *delbrueckii, Lactobacillus delbrueckii* subspecies *bulgaricus* type 2038, *Lactobacillus acidophilus* SBT-2062, *Lactobacillus salivarius* UCC 118, *Lactobacillus paracasei ST11,* and *Lactobacillus paracasei* subsp *paracasei* F19. *Lactococcus* species include, without limitation, *Lactococcus lactis* and *Lactococcus plantarum. Bacillus* species include, without limitation, *Bacillus subtilis. Bifidobacterium* species include, without limitation, *Bifidobacterium adolescentis, Bifidobacterium bifidum, Bifidobacterium animalis, Bifidobacterium thermophilum, Bifidobacterium breve, Bifidobacterium longum, Bifidobacterium pseudolongum, Bifidobacterium infantis* and *Bifidobacterium lactis. Saccharomyces* species include, without limitation, *Saccharomyces boulardii* (*cerevisiae*).

In one preferred embodiment, the compositions of the invention are pet or animal food compositions. These will advantageously include foods intended to supply necessary dietary requirements, as well as treats (*e*.*g*., biscuits) or other dietary supplements. Optionally, the pet or animal food compositions can be a dry composition (for example, kibble), semi-moist composition, wet composition, or any mixture thereof. The compositions are formulated for consumption by a feline animal, including but not limited to a domestic cat.

In another preferred embodiment, the composition is a dietary supplement, such as a gravy, drinking water, beverage, liquid concentrate, yogurt, powder, granule, paste, suspension, chew, morsel, treat, snack, pellet, pill, capsule, tablet, or any other delivery form. The dietary supplements can be specially formulated for consumption by a feline animal. In one detailed embodiment, the dietary supplement can comprise a high concentration of probiotic organisms such that the supplement can be administered to the animal in small amounts, or in the alternative, can be diluted before administration to an animal. The dietary supplement may require admixing with water prior to administration to the animal.

The composition may be refrigerated or frozen. The probiotic organisms may be preblended with the other components of the composition to provide the beneficial amounts needed, may be coated onto a pet food composition, dietary supplement, or food product, or may be added to the composition prior to offering it to the animal, for example, using a powder or a mix.

The compositions of the invention comprise probiotic organisms in an amount effective to enhance vaccine efficacy in an animal to which the composition has been administered. Pet foods can be formulated to contain probiotic organisms in the range of about 10² to about 10¹¹ colony forming units (CFU) per gram of the composition. Dietary supplements may be formulated to contain several fold higher concentrations of probiotic organisms, to be amenable for administration to an animal in the form of a tablet, capsule, liquid concentrate, or other similar dosage form, or to be diluted before administrations, such as by dilution in water, spraying or sprinkling onto a pet food, and other similar modes of administration.

In one example, the concentration of probiotic organisms in the composition is a function of the amount required to modulate immune functions, including an increase in the proportion and/or numbers of CD4+ lymphocytes in the blood of the animal. In an embodiment, the concentration of probiotic organisms in the composition is a function of an amount required to increase the concentration of immunoglobulins reactive against antigens of a specified pathogen in the blood serum, feces, secretions such as milk, tears, and saliva. The level of CD4+ lymphocytes and the concentration of immunoglobulins in the blood serum, feces, secretions such as milk, tears, and saliva of the animal may be determined by any means recognized and appreciated by one of skill in the art.

The compositions of the invention can optionally comprise supplementary substances such as minerals, vitamins, salts, condiments, colorants, and preservatives. Non-limiting examples of supplementary minerals include calcium, phosphorous, potassium, sodium, iron, chloride, boron, copper, zinc, magnesium, manganese, iodine, selenium and the like. Non-limiting examples of supplementary vitamins include vitamin A, various B vitamins, vitamin C, vitamin D, vitamin E, and vitamin K. Additional dietary supplements may also be included, for example, niacin, pantothenic acid, inulin, folic acid, biotin, amino acids, and the like.

The compositions of the invention can optionally comprise one or more supplementary substances that promote or sustain a healthy immune system, or further modulate immunity. Such substances include, without limitation, L-arginine, steroids such as 7-oxo dehydroepiandrosterone (7-oxo DHEA), carotenoids such as alpha- and beta-carotene, antioxidants, and herbs or herbal extracts such as astragalus and echinacea.

In various embodiments, animal food or dietary supplement compositions of the invention can comprise, on a dry matter basis, from about 15% to about 50% crude protein, by weight of the composition. The crude protein material may comprise vegetable proteins such as soybean, cottonseed, and peanut, or animal proteins such as casein, albumin, and meat protein. Non-limiting examples of meat protein useful herein include pork, lamb, equine, poultry, fish, and mixtures thereof.

The compositions may further comprise, on a dry matter basis, from about 5% to about 40% fat, by weight of the composition. The compositions may further comprise a source of carbohydrate. The compositions may comprise, on a dry matter basis, from about 15% to about 60% carbohydrate, by weight of the composition. Non-limiting examples of such carbohydrates include grains or cereals such as rice, corn, sorghum, alfalfa, barley, soybeans, canola, oats, wheat, and mixtures thereof. The compositions may also optionally comprise other materials such as dried whey and other dairy by-products.

The compositions may also comprise at least one fiber source. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide additional to the short chain oligofructose, mannanoligofructose, soy fiber, fiber from lupins, arabinogalactan, galactooligosaccharide, arabinoxylan, or mixtures thereof. Alternatively, the fiber source can be a fermentable fiber. Fermentable fiber has previously been described to provide a benefit to the immune system of companion animals. Fermentable fiber or other compositions known to those of skill in the art which provide a prebiotic composition that could enhance the growth of probiotic microorganisms within the intestine may also be incorporated into the composition to aid in the enhancement of the benefit provided by the present invention to the immune system of an animal.

In a detailed embodiment, the composition is a complete and nutritionally balanced pet or animal food. In this context, the pet food may be a wet food, a dry food, or a food of intermediate moisture content, as would be recognized by those skilled in the art of pet food formulation and manufacturing. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15%, and therefore is presented, for example, as small biscuit-like kibbles. The compositions and dietary supplements may be specially formulated for adult animals, or for older or young animals, for example, a "kitten chow," or "senior" formulation. In general, specialized formulations will comprise energy and nutritional requirements appropriate for animals at different stages of development or age.

Certain aspects of the invention are preferably used in combination with a complete and balanced food (for example, as described in National Research Council, 2006, Nutritional Requirements for Dogs and Cats, National Academy Press, Washington D.C., or Association of American Feed Control Officials, Official Publication 1996). That is, compositions comprising probiotic organisms according to certain aspects of this invention are preferably used with a high-quality commercial food. As used herein, "high-quality commercial food" refers to a diet manufactured to produce the digestibility of the key nutrients of 80% or more, as set forth in, for example, the recommendations of the National Research Council above for dogs and cats, or in the guidelines set forth by the Association of American Feed Control Officials. Similar high nutrient standards would be used for other animals.

The skilled artisan will understand how to determine the appropriate amount of probiotic organisms to be added to a given composition. Such factors that may be taken into account include the type of composition (*e*.*g*., pet food composition, dietary supplement, or food product), the average consumption of specific types of compositions by different animals, and the manufacturing conditions under which the composition is prepared. The concentrations of probiotic organisms to be added to the composition can be calculated on the basis of the energy and nutrient requirements of the animal. According to certain aspects, the probiotic organisms can be added at any time during the manufacture and/or processing of the composition. This includes, without limitation, as part of the formulation of the pet food composition, dietary supplement, or food product formulated, or as a coating applied to the pet food composition, dietary supplement, or food product.

The compositions can be made according to any method suitable in the art such as, for example, that described in Waltham Book of Dog and Cat Nutrition, Ed. ATB Edney, Chapter by A. Rainbird, entitled "A Balanced Diet" in pages 57 to 74, Pergamon Press Oxford.

### Methods

Described are methods for enhancing vaccine efficacy in a feline animal comprising administering to the animal a composition comprising one or more probiotic organisms in an amount effective to enhance vaccine efficacy in the animal. In some examples, the vaccine is for feline panleukopenia virus, feline rhinotracheitis virus, or feline calcivirus.

In detailed examples, the composition is a pet or animal food composition, dietary supplement, or food product as exemplified herein. In a further detailed example, the probiotic organisms include at least one of *Enterococcus spp*., preferably *E. faecium,* most preferably strain SF68, alone or combined with another probiotic organism, including one or more *Streptococcus spp., Lactobacillus spp., Lactococcus spp., Bacillus spp., Bifidobacterium spp., or Saccharomyces spp*., as described above.

The compositions can be administered to the animal by any of a variety of alternative routes of administration. Such routes include, without limitation, oral, intranasal, intravenous, intramuscular, intragastric, transpyloric, subcutaneous, rectal, and the like. In accordance with the claimed invention, the compositions are administered orally. As used herein, the term "oral administration" or "orally administering" means that the animal ingests or a human is directed to feed, or does feed, the animal one or more of the inventive compositions described herein.

Wherein the human is directed to feed the composition, such direction may be that which instructs and/or informs the human that use of the composition may and/or will provide the referenced benefit, for example, the enhancement of vaccine efficacy in the animal. Such direction may be oral direction (*e*.*g*., through oral instruction from, for example, a physician, veterinarian, or other health professional, or radio or television media (*i*.*e*., advertisement), or written direction (*e*.*g*., through written direction from, for example, a physician, veterinarian, or other health professional (*e*.*g*., prescriptions), sales professional or organization (*e*.*g*., through, for example, marketing brochures, pamphlets, or other instructive paraphernalia), written media (*e*.*g*., internet, electronic mail, or other computer-related media), and/or packaging associated with the composition (*e*.*g*., a label present on a container holding the composition).

Administration can be on an as-needed or as-desired basis, for example, once-monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal or otherwise contacted with or admixed with daily feed or food. When utilized as a daily feed or food, administration will be well known to those of ordinary skill.

Administration can also be carried out on a regular basis, for example, as part of a diet regimen in the animal. A diet regimen may comprise causing the regular ingestion by the animal of a composition comprising one or more probiotic organisms in an amount effective to modulate immunity or to enhance vaccine efficacy in the animal. Regular ingestion can be once a day, or two, three, four, or more times per day, on a daily or weekly basis. Similarly, regular administration can be every other day or week, every third day or week, every fourth day or week, every fifth day or week, or every sixth day or week, and in such a dietary regimen, administration can be multiple times per day. The goal of regular administration is to provide the animal with the preferred daily dose probiotic organisms, as exemplified herein.

The daily dose of probiotic organisms can be measured in terms of colony forming units (CFU) administered per animal, per day. The daily dose of probiotic organisms can range from about 10⁵ to about 10¹² CFU/day. More preferably, the daily dose of probiotic organisms is about 10⁷ to about 10⁹ CFU/day. More preferably, the daily dose of probiotic organisms is about 10⁸ to about 10⁹ CFU/day. Most preferably, the daily dose of probiotic organisms is about 10⁸ CFU/day.

Administration of the compositions comprising one or more probiotic organisms, including administration as part of a diet regimen, can span a period of time ranging from gestation through the entire life of the animal.

The following examples are provided to describe the invention in greater detail. They are intended to illustrate, not to limit, the invention.

### Example 1

### Animals and Experimental Parameters

**Feline study population.** Twenty, six-week old SPF kittens were purchased from a Liberty Laboratories (Liberty, NY). The kittens were shown to be seronegative for feline leukemia virus antigen and feline immunodeficiency virus antibodies by ELISA. (Snap Combo, IDEXX Laboratories, Portland, ME).

**Experimental design.** After a 10 day equilibration period, the kittens were randomized into two groups of ten kittens each and the treatment study started at 7 weeks of age. Between 0.25 and 0.28 g (∼5 x 10⁹ CFU based on dilution count assays) of LBC ME5 PET *E. faecium* SF68 (NCIMB 10415) (Cerbios-Pharma SA, Switzerland) were added into individual 50 mL conical bottom polypropylene centrifuge tubes, capped, and stored at 4°C for the duration of the study. Similar preparations were used for aliquots of the palatability enhancer (a typical pet food coating comprising liver digest as the main component was used) using 150 mg per tube. Aliquots were monitored for water absorption and were to be discarded if there appeared to be any clumping of either the probiotic or palatability enhancer. Just before administration, one aliquot of palatability enhancer was transferred to one of the stored *E. faecium* SF68 tubes (treatment group) or an empty tube (placebo group) and diluted using room temperature tap water to a total volume of 10 mL. Contents were vortexed for at least three minutes and aspirated into a 12 cc syringe. Immediately after vortexing the suspension, appropriate kittens were orally administered 1 ml of either the *E. faecium* SF68 (total daily dose 5x10⁸ CFU per day) or the palatability enhancer alone (placebo kittens) until they were 27 weeks of age. Both groups were fed dry kitten food *ad libitum* (typical kitten growth formula meeting all AAFCO requirements and was based on chicken and rice as main ingredients was used) and gang housed in two separate rooms to avoid cross-contamination with the probiotic. At 9 and 12 weeks of age, all kittens were vaccinated subcutaneously with a modified live combination vaccine (Pfizer Animal Health, Exton, PA) for feline herpesvirus-1, calicivirus, and panleukopenia virus as recommended by the American Association of Feline Practitioners. (Richards J *et al.* (2001)).

Statistical evaluation. On each sample date, group mean values for all measured parameters were calculated. Differences between the probiotic-treated group and placebo group were analyzed using a mixed ANOVA model appropriate for a repeated measures experiment. Time was included in the model as a continuous variable. Percentages of cat samples positive for *C*. *perfringens* enterotoxin or *C. difficile* toxins A or B and percentages of gated cells positive for cell surface markers were calculated for each group of cats over the duration of the study and compared by a two tailed t test. (GRAPHPAD Prism, GRAPHPAD Software, Inc., San Diego, CA). Statistical significance was considered to be p < 0.05.

### Example 2

### Sample Collection and Clinical Monitoring

The attitudes and behavior of the kittens were monitored daily throughout the study. Body weight was measured weekly. Blood, saliva, and feces were collected from all cats prior to starting probiotic or palatability enhancer supplementation at 7 weeks of age and at 9, 15, 21, and 27 weeks of age. In addition, feces were collected from kittens in the treatment group at 28 weeks of age. For each group of kittens, 5 fecal samples per day were randomly selected from the shared litterbox and scored using a standardized graphic scoring card and the daily group means determined. Fecal extracts for total IgA and total IgG measurement were processed according to the protocol described by Benyacoub J *et al.* (2003)). All samples were stored at -80°C until assayed in batches.

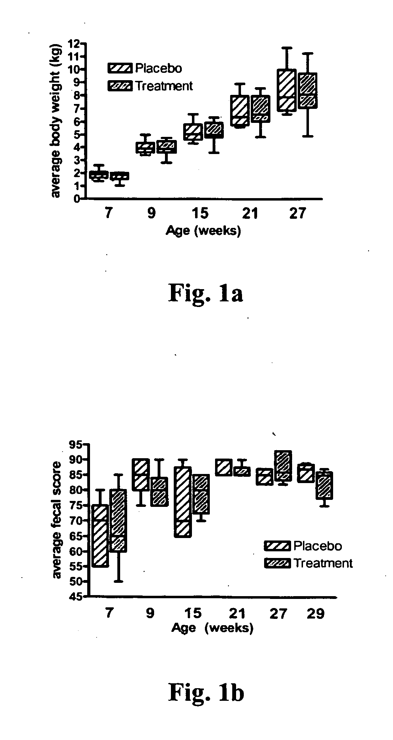

The stools of all kittens were normal at the beginning of the supplementation period (7 weeks of age). One kitten in each group was removed from the study for reasons unrelated to the study and were therefore removed from the final data analysis. Body weight and fecal scores were not statistically different between the two groups over time or at any individual time points (Figure 1).

Complete blood cell counts, biochemistry parameters, and body weights were similar between groups of cats over the course of the study. Fecal scores were similar between groups as well suggesting that use of SF68 at the dosage described here will induce no noticeable clinical abnormalities.

### Example 3

### Fecal Assays

On each sample date, feces from each kitten were plated in eight serial 10-fold dilutions onto KF Streptococcus Agar and incubated for 48 hours at 37°C aerobically. Ten colonies of each morphology type were picked off using sterile loops and placed in 1.2 mL brain heart infusion medium (BHI) (Becton Dickinson, Franklin Lakes, NJ) and stored at - 80°C pending analysis. RAPD-PCR was performed on bacterial isolates from each sample to determine if viable *E. faecium* SF68 was in the stools of treated cats and to assess whether the probiotic was accidentally transmitted from the treated kittens to the control kittens. The thermocycler parameters were as follows: 30 cycles of one minute of denaturation at 95°C, one minute of annealing 40°C, four minutes extension at 72°C. The 25.5 µL reaction mixture included 2.45 µL 10x magnesium-free buffer (100 mM Tris-HCl, pH 8.3, 500 mM KCl), 3.22 mM MgCl2, 0.4 µL (1 Unit), JumpStart Taq DNA polymerase (Sigma D-4184, Sigma-Aldrich, Inc., St. Louis, MO), 1.9 µL dNTP mix (2.5 mM), 1 µL primer (100 uM), 15.47 µL PCR water, and 1 µL bacterial culture. The sequence of the primer used was 5'-GGTTGGGTGAGAATTGCACG-3'. Five to ten µL of the PCR product was run on a two percent agarose gel and patterns of banding were compared to a positive SF68 control. Commercially available ELISAs were used to determine whether Clostridium perfringens enterotoxins or *C.* difficile toxins A/B were present in the feces of all kittens. (*C. perfringens* (ELISA, Kit No. 92-000-22) and *C. difficile* (ELISA, Kit No. 94-0150-KT), Techlabs, Blacksburg, VA.) Routine aerobic fecal cultures for *Salmonella* spp. and *Campylobacter* spp. were performed by the Colorado State University Diagnostic Laboratory.

Feces from seven of nine treatment cats were positive for SF68 on at least one time point during the study. However, SF68 DNA was not amplified from feces of any treated cat 1 week after stopping supplementation (week 28). Neither *Salmonella* spp. nor *Campylobacter* spp. were grown from feces. All samples from placebo cats were negative for SF68 by RAPD PCR. Numbers of positive samples for *C. difficile* toxins A/B or *C. perfringens* enterotoxin (Table 1) did not vary between the groups over the course of the study.

*Salmonella spp.* and *Campylobacter spp.* shedding was not induced by SF68 supplementation. Several fecal samples in both groups of kittens were positive for *C. difficile* or *C. perfringens* toxins; however, there was no significant difference in number of positive samples between groups and positive results did not correlate to the presence of diarrhea. SF68 was detected in the feces of the majority of treated cats during the period of supplementation, but was no longer detected in the feces 1 week after stopping supplementation indicating that the organism persisted in the cats only transiently. Thus, administration of SF68 using the dosage described herein has no deleterious effects and is safe for administration in the time period studied.

### Example 4

### Immunologic Assays

Complete blood counts, serum biochemical panels, and urinalyses were performed at the Clinical Pathology Laboratory at Colorado State University. Antigen specific humoral immune responses were estimated by measuring serum FHV-1-specific IgG,FHV-1-specific IgA, FCV-specific IgG, and feline panleukopenia-specific IgG10 in sera as well as FHV-1 specific IgG and IgA levels in saliva using adaptations of previously published ELISA assays. (Lappin MR *et al.* (2002); and Ditmer DA *et al.* (1998). For FHV-1 specific IgG and IgA, results were calculated by both the mean absorbance for the triplicate test wells for each sample and by calculation of percentage ELISA units (test sample mean absorbance minus the negative control sample mean absorbance/positive control sample mean absorbance minus the negative control sample mean absorbance multiplied by 100). For FCV and FPV, mean absorbances were used. Total IgG and IgA concentrations in sera, fecal extracts, and saliva were estimated by use of commercially available ELISA assays or radial immunodiffusion assay. (Bethyl Laboaratories, Inc., Montgomery, TX).

Cellular immune responses were assessed via flow cytometry and whole blood proliferation assays. Flow cytometry was performed within 12 hours of blood collection using 500 µL of anticoagulated (EDTA) blood incubated at room temperature in red cell lysis buffer (0.155 M NH₄Cl/0.010 MKHCO₃/5 X 10⁻⁴% Phenol Red (0.5%). Cells were washed two times with PBS and the resultant cell pellets were resuspended in FACS buffer containing PBS, 0.1% sodium azide and 2% fetal bovine serum to attain a concentration of 1x10⁶ cells/100 µL if possible. Samples with insufficient cells for at least 500 µL of the above suspension were counted and cell concentration recorded. One hundred µL of each cell suspension was added to individual wells in a round-bottom 96 well plate for immunostaining. Non-specific binding was blocked by addition of 10% normal cat serum. (Jackson ImmunoResearch Laboratories, Inc., West Grove, PA). Immunostaining was done at 4°C in the dark in FACS buffer. Lymphocytes were stained for expression of CD4 and CD8 (vpg34; anti-CD4-fitc, vpg9; anti-CD8-rpe antibodies; Serotec, Raleigh, NC (Oxford, UK)) and expression of CD44 (IM7; anti-CD44-pe/cy5 antibody; Pharmingen, Franklin Lakes, NJ). For analysis of B cells, lysed whole blood was immunostained with cross-reactive antibodies to B220 (ra3-b62; anti-B220-biotinylated antibody; eBioscience, San Diego, CA), CD21(b-ly4; anti-CD21-apc antibody; BD-Biosciences, Franklin Lakes, NJ), and MHC class II (anti-MHC class II-fitc antibody; clone CAG5-3D1, Serotec, Raleigh, NC (Oxford, UK)). Cells for analysis were gated on live lymphocyte populations based on forward and side-scatter characteristics. Data were collected on a Cyan MLE cytometerp and analyzed using Summit software. (Dako-Cytomation, Fort Collins, CO).

Proliferation assays were performed in triplicate using 10 µL whole heparinized blood preconditioned by incubating in 100 µL complete tumor media at 37°C with 5% CO2 for 30 minutes before addition of the mitogen or antigen. (Complete tumor media: modified Eagle's medium supplemented with essential and non-essential amino acids + 10% FBS). Cells were maintained in medium alone (unstimulated), or stimulated with concanavalin A (10 µg/mL: Con ASigma-Aldrich, St. Louis, MO), or a FHV-1 antigen preparation (1 µL/well, prepared prior to the start of the study and stored aliquotted at -80°C) for 96 hours at 37°C with 5% CO2. (Veir JK *et al*. (2005)). Cells were pulsed with 1 µCi tritiated thymidine per well and harvested 18 hours later onto fiberglass filter mats. (Wallac-Microbeta Perkin -Elmer, Boston, MA). Mats were read using a MicroBetas liquid scintillation counter. The mean stimulation index (mean maximum count per stimulated sample divided by mean maximum count per unstimulated sample) was calculated for all samples.

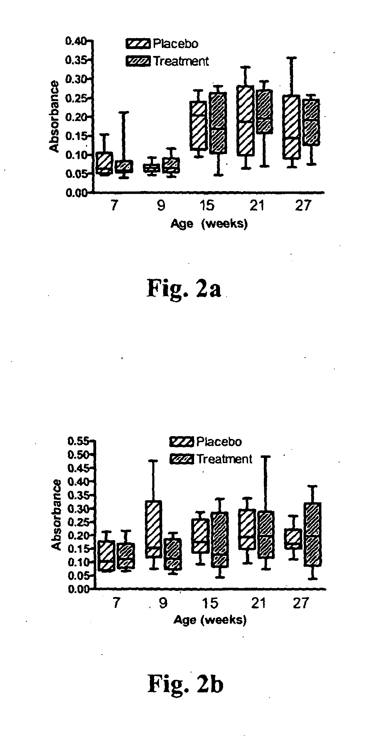

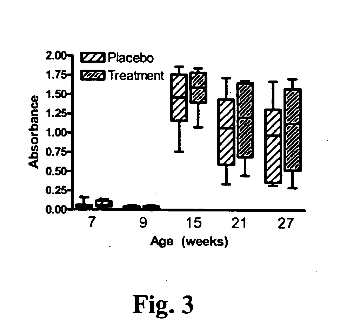

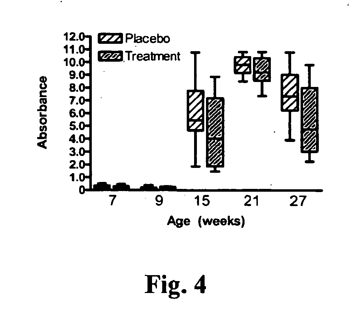

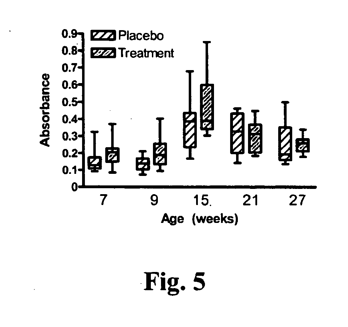

Complete blood counts and biochemical profiles were within normal limits for the age group for all cats at all time points. There was no statistical difference between the groups over time or at any individual time points among the assays analyzed. At 21 and 27 weeks of age, the mean levels of FHV-1-specific IgA in serum and saliva were numerically greater in the treatment group when compared to the placebo group, but the differences were not statistically different (Figure 2). At 15, 21, and 27 weeks of age the mean FHV-1-specific serum IgG levels were numerically greater in the treatment group when compared to the placebo group using both assays, but the differences were not statistically significantly different (Figure 3). No FHV-1 specific IgG was detected in saliva. FCV-specific-IgG levels in serum were similar between groups (Figure 4). At 15 weeks of age, the treatment group serum mean FPV-specific IgG levels were numerically greater than those of the placebo group, but the differences were not statistically significantly different (Figure 5).

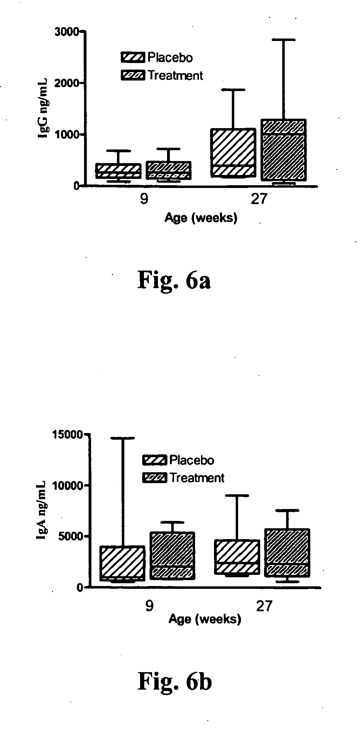

Concentrations of total IgG and IgA in serum were similar between groups (data not shown). Total IgG was not detected in saliva and total IgA concentrations in saliva were similar between groups (data not shown). At 27 weeks of age, the treatment group mean concentrations of total IgG in fecal extracts were numerically greater than those of the placebo group, but the differences were not statistically different (Figure 6). Total IgA concentrations in fecal extracts were similar between groups (Figure 6).

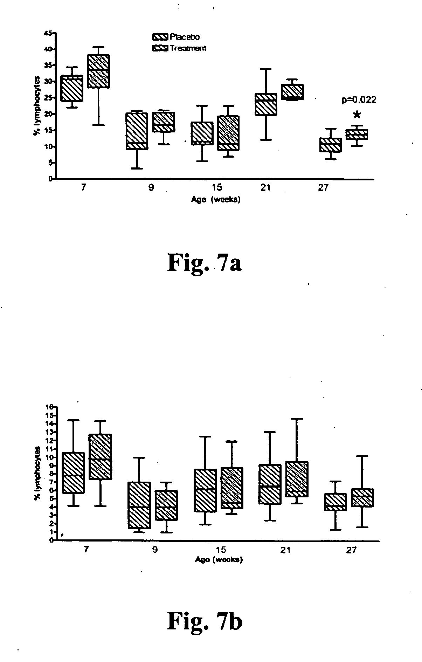

Proliferation assays using either 10 µg/mL concanavalin A or 1 µL FHV-1 antigen preparation as the stimulants did not produce significantly different mean maximum counts between groups at any time points. There were no statistical differences between the groups for any cell surface markers at the first four time points (Figure 7). At 27 weeks of age, the treatment group (mean 13.87%) had a significantly higher percentage of gated lymphocytes positive for CD4 than the placebo group (mean 10.61 %, p = 0.0220). No other comparisons were significantly different.

The increase in CD4+ counts in the treatment group compared to the placebo group without a concurrent increase in CD8+ counts at 27 weeks of age demonstrates systemic immune modulating effects by the probiotic. The detection of numerically greater humoral immune response parameters at some collection times suggests that stimulation of Th1 responses occurred. This hypothesis is supported by the findings of the study of SF68 supplementation in puppies. (Benyacoub J *et al.* (2003)).

After vaccinations, each of the kittens developed FHV-1, FCV, and FPV-specific serum antibody responses that are similar to other studies indicating they were immunocompetent and that the modified live vaccine used was viable. (Lappin MR *et al.* (2002)). Several of the results also indicate that feeding of the probiotic influenced humoral and cell-mediated immune responses of these kittens. These include the detection of statistically significantly greater CD4+ lymphocytes counts at 27 weeks of age and numerically greater mean values for FHV-1-specific IgA in serum and saliva at 21 and 27 weeks of age, FHV-1-specific IgG levels in serum at 15, 21, and 27 weeks of age, FPV-specific IgG levels in serum at 15 weeks of age, and total IgG concentrations in fecal extracts at 27 weeks of age when compared to the placebo group.

### References

Benyacoub J, Czarnecki-Maulden GL, Cavadini C, Sauthier T, Anderson RE, Schiffrin EJ, von der WT (2003), Supplementation of food with Enterococcus faecium (SF68) stimulates immune functions in young dogs. J. Nutr.133: 1158-1162
Ditmer DA, Lappin MR, Carman J, Collins JK (1998), Enzyme-linked immunosorbent assay for detection of feline herpesvirus 1 IgG in the serum, aqueous humor, and cerebrospinal fluid. J. Vet. Diagn. Invest. 10: 315-319
Drisko JA, Giles CK, Bischoff BJ (2003), Probiotics in health maintenance and disease prevention. Altern. Med. Rev. 8:143-155
Isolauri E, Sutas Y, Kankaanpaa P, Arvilommi H, Salminen S (2001 a), Probiotics: effects on immunity. Am. J. Clin. Nutr. 73: 444S
Isolauri E (2001b), Probiotics in human disease. Am. J. Clin. Nutr. 73: 1142S-1146S
Kim LM, Morley PS, Traub-Dargatz JL, Salman MD, Gentry-Weeks C (2001), Factors associated with Salmonella shedding among equine colic patients at a veterinary teaching hospital. J. Am. Vet. Med. Assoc. 218: 740-748
Lappin MR, Andrews J, Simpson D (2002), Use of serologic tests to predict resistance to feline herpesvirus 1, feline calicivirus, and feline parvovirus infection in cats. J. Am. Vet. Med. Assoc. 220: 38-42
Macpherson AJ, Uhr T (2004), Induction of protective IgA by intestinal dendritic cells carrying commensal bacteria, Science 303: 1662-1665
Oyetayo VO and Oyetayo FL (2004), Potential of probiotics as biotherapeutic agents targeting the innate immune system. Afr. J. Biotechnol. 4:123-127.
Reid G, Jass J, Sebulsky MT, McCormick JK (2003), Potential uses of probiotics in clinical practice. Clin. Microbiol. Rev. 16:658-672
Richards J, Rodan I, Elston T, Flemmin D, Ford R, Henry S, Hustead D, Lappin MR, Paul M, Rosen D, Scherk M, Scott F, Welborn L (2001). Feline Vaccine Selection and Administration, Compend. Cont. Ed. Pract. Vet. 23: 71-80
Sanders ME (2003), Probiotics: considerations for human health Nutr. Rev. 61: 91-99
Schrezenmeir J, de Vrese M (2001), Probiotics, prebiotics, and synbiotics--approaching a definition. Am. J. Clin Nutr. 73: 361 S
Scott FW, Geissinger CM (1999), Long-term immunity in cats vaccinated with an inactivated trivalent vaccine. Am. J. Vet. Res. 60: 652-8
Slater E, York C (1976), Comparative studies on parenteral and intranasal inoculation of an attenuated feline herpes virus. Dev. Biol. Stand. 33: 410-6
Sykes JE, Anderson GA, Studdert VP, Browning GF (1999), Prevalence of feline Chlamydia psittaci and feline herpesvirus 1 in cats with upper respiratory tract disease. J. Vet. Intern. Med. 13: 153-162
Van Pelt DR, Lappin MR (1994), Pathogenesis and treatment of feline rhinitis. [Review] [48 refs]. Veterinary Clinics of North America - Small Animal Practice. 24: 807-823
Veir JK, Lappin MR, Radecke S. Whole blood proliferation assay to assess cell mediated immune responses to FHV-1. J. Vet. Diagn. Invest. 2005. (In Press)

## Claims

1. Use of a probiotic *Enterococcus* bacteria, administered orally, in the preparation of a medicament for enhancing the vaccine efficacy of a FVH-1, FCV or FPV vaccine in a feline animal.

2. Use according to claim 1 wherein the medicament is a food composition or dietary supplement.

3. Use according to claim 1 or 2 wherein the probiotic *Enterococcus* is *E. faecium* strain SF68.

4. Use according to any preceding claim, wherein the probiotic *Enterococcus* is present in an amount of at least about 10² to about 10¹¹ colony forming units (CFU) per gram of the formulation.

5. Use according to any preceding claim wherein the medicament further comprises 7-oxo-dehydroepiandrosterone (7-oxo-DHEA).

6. Use according to any preceding claim, wherein the medicament further comprises at least one other type of probiotic organism.

7. Use according to any preceding claim, wherein the feline animal is a domestic cat.

8. Use according to any one of the preceding claims wherein enhancing vaccine efficacy in the animal results in increased production of CD4+ lymphocytes in the animal.

9. Use according to any one of the preceding claims wherein enhancing vaccine efficacy in the animal results in increased concentration of immunoglobulins reactive against antigens of a specified pathogen in the blood serum, feces, milk, tears, saliva, respiratory epithelium, or gastrointestinal epithelium of the animal.

10. A composition comprising one or more probiotic *Enterococcus* bacteria, administered orally, for use in enhancing the vaccine efficacy of a FVH-1, FCV or FPV vaccine in a feline animal.

11. The composition of claim 10, wherein the composition is an animal food composition or dietary supplement.

12. The composition of claim 10 or 11, wherein the probiotic *Enterococcus* is *E. faecium* strain SF68.

13. The composition of any one of claims 10 to 12, wherein the probiotic *Enterococcus* is present in an amount of at least about 10² to about 10¹¹ CFU per gram of the formulation.

14. The composition of any one of claims 10 to 13, wherein the feline animal is a domestic cat.

15. The composition of any one of claims 10 to 14, wherein enhancing vaccine efficacy in the animal results in increased production of CD4+ lymphocytes in the animal.

16. The composition of any one of claims 10 to 15, wherein enhancing vaccine efficacy in the animal results in increased concentration of immunoglobulins reactive against antigens of a specified pathogen in the blood serum, feces, milk, tears, saliva, respiratory epithelium, or gastrointestinal epithelium of the animal.

## Patentansprüche

1. Verwendung eines probiotischen *Enterococcus* Bakteriums, das oral verabreicht wird, für die Herstellung eines Medikaments zur Steigerung der Impfwirksamkeit eines FVH-1, FCV oder FPV Impfstoffs in einer Katze.

2. Verwendung nach Anspruch 1 wobei das Medikament eine Nahrungsmittelzusammensetzung oder Nahrungsergänzungsmittel ist.

3. Verwendung nach Anspruch 1 oder 2, wobei der probiotische *Enterococcus* der *E. faecium* Stamm SF68 ist.

4. Verwendung nach einem der vorstehenden Ansprüche, wobei der probiotische *Enterococcus* in einer Menge von mindestens ungefähr 10² bis ungefähr 10¹¹ Kolonie bildender Einheiten (KBE) pro Gramm der Formulierung vorhanden ist.

5. Verwendung nach einem der vorstehenden Ansprüche, wobei das Medikament weiterhin 7-Oxo-dehydroepiandrosteron (7-oxo-DHEA) umfasst.

6. Verwendung nach einem der vorstehenden Ansprüche, wobei das Medikament weiterhin mindestens einen weiteren probiotischen Organismus umfasst.

7. Verwendung nach einem der vorstehenden Ansprüche, wobei die Katze eine Hauskatze ist.

8. Verwendung nach einem der vorstehenden Ansprüche, wobei die Steigerung der Impfwirksamkeit in dem Tier zu einer erhöhten Produktion von CD4+ Lymphozyten in dem Tier führt.

9. Verwendung nach einem der vorstehenden Ansprüche, wobei die Steigerung der Impfwirksamkeit in dem Tier zu einer erhöhten Konzentration von Immunglobulinen führt, die gegen Antigene eines spezifischen Pathogens in dem Blutserum, dem Fäzes, der Milch, den Tränen, dem Speichel, dem respiratorischem Epithel oder dem Magen-Darm-Epithel des Tiers reagieren.

10. Zusammensetzung umfassend, ein oder mehrere oral verabreichte, probiotische *Enterococcus* Bakterien, die verwendet werden, um die Impfwirksamkeit eines FVH-1, FCV oder FPV Impfstoffs in einer Katze zu steigern.

11. Zusammensetzung nach Anspruch 10, worin die Zusammensetzung eine Nahrungsmittelzusammensetzung oder ein Nahrungsergänzungsmittel ist.

12. Zusammensetzung nach Anspruch 10 oder 11, worin der probiotische *Enterococcus* der *E. faecium* Stamm SF68 ist.

13. Zusammensetzung nach einem der Ansprüche 10 bis 12, worin der probiotische *Enterococcus* in einer Menge von mindestens ungefähr 10² bis ungefähr 10¹¹ KBE pro Gramm der Formulierung vorhanden ist.

14. Zusammensetzung nach einem der Ansprüche 10 bis 13, worin die Katze eine Hauskatze ist.

15. Zusammensetzung nach einem der Ansprüche 10 bis 14, worin die Steigerung der Impfwirksamkeit in dem Tier zu einer erhöhten Produktion von CD4+ Lymphozyten in dem Tier führt.

16. Zusammensetzung nach einem der Ansprüche 10 bis 15, worin die Steigerung der Impfwirksamkeit in dem Tier zu einer erhöhten Konzentration von Immunglobulinen führt, die gegen Antigene eines spezifischen Pathogens in dem Blutserum, dem Fäzes, der Milch, den Tränen, dem Speichel, dem respiratorischen Epithel oder dem Magen-Darm-Epithel des Tiers reagieren.

## Revendications

1. Utilisation d'une bactérie *Enterococcus* probiotique, administrée par voie orale, dans la préparation d'un médicament destiné à augmenter l'efficacité de vaccin d'un vaccin contre le FVH-1, FCV ou FPV chez un félin.

2. Utilisation suivant la revendication 1, dans laquelle le médicament est une composition alimentaire ou un supplément diététique.

3. Utilisation suivant la revendication 1 ou 2, dans laquelle l'Enterococcus probiotique est la souche de *E. faecium* SF68.

4. Utilisation suivant l'une quelconque des revendications précédentes, dans laquelle l'Enterococcus probiotique est présent en une quantité d'au moins environ 10² à environ 10¹¹ unités de formation de colonies (CFU) par gramme de la formulation.

5. Utilisation suivant l'une quelconque des revendications précédentes, dans laquelle le médicament comprend en outre de la 7-oxo-déhydroépiandrostérone (7-oxo-DHEA).

6. Utilisation suivant l'une quelconque des revendications précédentes, dans laquelle le médicament comprend en outre au moins un autre type d'organisme probiotique.

7. Utilisation suivant l'une quelconque des revendications précédentes, dans laquelle le félin est un chat domestique.

8. Utilisation suivant l'une quelconque des revendications précédentes, dans laquelle l'augmentation de l'efficacité de vaccin chez l'animal a pour résultat une production accrue de lymphocytes CD4+ chez l'animal.

9. Utilisation suivant l'une quelconque des revendications précédentes, dans laquelle l'augmentation de l'efficacité de vaccin chez l'animal a pour résultat une concentration accrue d'immunoglobulines réactives contre des antigènes d'un agent pathogène spécifié dans le sérum sanguin, les matières fécales, le lait, les larmes, la salive, l'épithélium respiratoire ou l'épithélium gastro-intestinal de l'animal.

10. Composition comprenant une ou plusieurs bactéries Enterococcus probiotiques administrées par voie orale, destinée à être utilisée pour augmenter l'efficacité de vaccin d'un vaccin contre le FVH-1, FCV ou FPV chez un félin.

11. Composition suivant la revendication 10, ladite composition étant une composition alimentaire ou un supplément diététique pour animaux.

12. Composition suivant la revendication 10 ou 11, dans laquelle l'Enterococcus probiotique est la souche de *E. faecium* SF68.

13. Composition suivant l'une quelconque des revendications 10 à 12, dans laquelle l'Enterococcus probiotique est présent en une quantité d'au moins environ 10² à environ 10¹¹ unités de formation de colonies (CFU) par gramme de la formulation.

14. Composition suivant l'une quelconque des revendications 10 à 13, dans laquelle le félin est un chat domestique.

15. Composition suivant l'une quelconque des revendications 10 à 14, dans laquelle l'augmentation de l'efficacité de vaccin chez l'animal a pour résultat une production accrue de lymphocytes CD4+ chez l'animal.

16. Composition suivant l'une quelconque des revendications 10 à 15, dans laquelle l'augmentation de l'efficacité de vaccin chez l'animal a pour résultat une concentration accrue d'immunoglobulines réactives contre les antigènes d'un agent pathogène spécifié dans le sérum sanguin, les matières fécales, le lait, les larmes, la salive, l'épithélium respiratoire ou l'épithélium gastro-intestinal de l'animal.
